# FASCICULE DE BREVET EUROPEEN

(11) **EP 1 135 110 B1**
(45) Date de publication et mention de la délivrance du brevet: **20.08.2003**
(21) Numéro de dépôt: 99972916.3
(22) Date de dépôt: 30.11.1999
(51) Int. Cl.: A61K 9/16, A61K 9/46

(54) **PREPARATION DE COMPOSITIONS SECHES SOLUBLES EN PRESENCE D'EAU ET EVITANT LA REACTION DE MAILLARD A L'ETAT SEC ET LEURS APPLICATIONS**
HERSTELLUNG TROCKENER, IN WASSER LÖSLICHER ZUSAMMENSETZUNGEN ZUR VERMEIDUNG DER MAILLARD-REAKTION IM TROCKENEN ZUSTAND UND IHRE ANWENDUNGEN
PREPARATION OF DRY COMPOSITIONS SOLUBLE IN THE PRESENCE OF WATER AND AVOIDING MAILLARD REACTION IN DRY STATE AND USES

(30) Priorité: 01.12.1998 FR 9815113
(43) Date de publication de la demande: 26.09.2001
(73) Titulaire: VIRBAC, 06516 Carros (FR)
(72) Inventeur: DERRIEU, Guy, F-06800 Cagnes sur Mer (FR); BROUSSAUD, Olivier, F-06300 Nice (FR); POUGNAS, Jean-Luc, F-06700 Saint Laurent du Var (FR)
(74) Mandataire: Goulard, Sophie
(86) Numéro de dépôt international: FR9902955
(87) Numéro de publication internationale: WO00032170

(56) Documents cités:
- EP-A- 0 153 998
- EP-A- 0 396 335
- DE-A- 4 416 769
- FR-M- 5 225

## Description

La présente invention concerne la préparation de compositions sèches solubles en présence d'eau, évitant la réaction de Maillard à l'état sec, et leurs applications comme médicament, produit alimentaire, produit diététique ou produit cosmétique.

La réaction entre le glucose et les protéines est connue depuis fort longtemps. La première observation en a été faite pendant la cuisson d'aliments par Louis Camille Maillard en 1911. Il a mis en évidence que le glucose ou tout autre sucre réducteur réagit avec un aminoacide pour conduire après des étapes de déshydratation et des réarrangements à des formes stables brunes. Plus généralement, la réaction de Maillard est une cascade de réactions chimiques initiées par des condensations réversibles spontanées d'un groupement carbonyle avec un groupement amino, pour former une base de Schiff. L'énergie nécessaire pour initier la condensation est seulement de l'ordre de 42 à 63 kJ. Cette réaction est réversible en présence d'eau, particulièrement en solution, où l'équilibre est largement en faveur des groupements réactifs. En revanche, les réarrangements d'Amadori décrits pour les amines de formule RR'NH ou d'Heyns décrits pour l'ammoniac (NH₃) et les amines monosubstituées (RNH₂), qui suivent immédiatement la formation de la base de Schiff, sont irréversibles et déclenchent une série de réactions qui conduisent à la production de composés bruns.

Dans l'industrie alimentaire, la réaction de Maillard a été très étudiée. Du fait que les produits de la réaction de Maillard ont une influence sur la couleur, la saveur et l'odeur des aliments, cette réaction est souvent exploitée lorsqu'elle conduit à des effets désirés. C'est notamment le cas dans le domaine de la brasserie où la réaction de Maillard peut être responsable à la fois de la couleur, de l'odeur et du goût de la bière.

Cependant cette réaction peut conduire à des transformations organoleptiques non désirées des aliments (goût rance, odeur ou goût de brûlé, couleur brune) et réduit la disponibilité des composés participant à la réaction.

Ainsi, cette réaction est une des causes de la détérioration des aliments pendant leur conservation, spécialement des produits séchés. Des dégradations ont même été observées sur des produits stockés dans des endroits réfrigérés. En effet, la réaction de Maillard empêche la conservation des compositions sèches associant des molécules possédant un groupement amino avec des molécules possédant un groupement carbonyle.

Aussi, des compositions réhydratantes, telle la composition décrite dans le brevet français n° 2 467 599, qui contient du glucose associé à au moins un acide aminé libre ou sous forme de sel et à un sel de sodium d'acide carboxylique, se sont révélées instables à l'état sec.

Puisqu'il est impossible de proposer des compositions sèches réhydratantes pour animaux, contenant du glucose et des aminoacides dans un même emballage, qui soient stables à l'état sec et solubles dans l'eau et puisque l'utilisation de glucose, de disaccharides et d'acides aminés, couplés à un apport adéquat d'électrolytes, est à la base de la confection de nombreux réhydratants synthétiques, ceux-ci sont présentés, soit dans des emballages distincts, soit dans des sachets bi-compartimentés (ENERLAC® réhydratant oral pour veau), le mélange étant alors réalisé au moment de la dissolution dans l'eau de boisson des animaux.

De tels emballages constituent une contrainte technique et économique importante et sont aussi la cause d'une mauvaise utilisation du produit, (médicament ou produit cosmétique, alimentaire ou diététique), soit par oubli d'un sachet contenant un des composants, soit par l'utilisation de deux sachets contenant le même composant.

Pour éviter la réaction de Maillard dans des compositions où elle est susceptible de se produire, une des solutions consiste à ajouter dans ces compositions des inhibiteurs de la glycosylation des protéines, comme par exemple des dérivés ou des diesters phosphates de l'acide ascorbique et du tocophérol (demande internationale WO 9640622 et demande de brevet européen EP 0430045).

Une autre stratégie visant à prévenir la disparition d'un composé portant des fonctions amino libres du fait de la réaction de Maillard consiste à ajouter dans la composition un substituant tel que la glycine ou ses sels qui participera à la réaction de Maillard au lieu du composé qui sera ainsi préservé (Brevet US 4,371,557).

Ces solutions présentent-néanmoins différents inconvénients, dont le principal est d'introduire dans la composition, un nouveau composant dont l'unique fonction est d'empêcher la réaction de Maillard. Ceci peut alourdir considérablement les formulations, ce qui n'est pas souhaitable, en particulier lorsqu'il s'agit de compositions pharmaceutiques. Par ailleurs, le composant que l'on ajoute réagit le plus souvent avec le sucre contenu dans la composition, lequel est alors rendu indisponible.

Une autre solution pour éviter la réaction de Maillard consiste à enrober les acides aminés de manière à empêcher toute réaction avec le sucre durant la conservation à l'état sec (demandes de brevets JP 58079962 et JP 57128662). Cet enrobage nécessite un important travail de mise en oeuvre et conduit à une disponibilité restreinte ou retardée de l'aminoacide.

Les Inventeurs se sont par conséquent fixé pour but de pourvoir à un procédé de préparation de compositions sèches solubles en présence d'eau, permettant d'éviter la réaction de Maillard dans lesdites compositions à l'état sec.

La présente invention a en conséquence pour objet, l'utilisation d'au moins une molécule (A) comprenant un ou plusieurs groupes amino protégés par des groupes protecteurs, lesdits groupes protecteurs s'éliminant en présence d'eau, en association avec au moins une molécule (B) portant un ou plusieurs groupes carbonyle pour éviter la réaction de Maillard dans des compositions sèches à l'état sec et solubles en présence d'eau.

De façon inattendue, les Inventeurs ont montré que l'utilisation de l'association, sous forme de composition sèche, d'au moins une molécule (A) comprenant un ou plusieurs groupes amino protégés par des groupes protecteurs qui s'éliminent en présence d'eau, avec au moins une molécule (B) portant un ou plusieurs groupes carbonyle, permet de conserver sous cette forme l'ensemble de ces constituants sans en altérer la qualité.

En présence d'eau, la ou les fonctions amines de la ou des molécules (A) comprenant un ou plusieurs groupes amino protégés par des groupes protecteurs sont libérées, et l'on obtient une solution contenant la ou les molécules (A) dont la ou les fonctions amines sont libres et ont été protégées de la réaction de Maillard pendant la conservation sous la forme sèche, et la ou les molécules (B).

Dans un mode de mise en oeuvre avantageux de l'utilisation selon l'invention, la ou les fonctions amines des molécules (A) peuvent être protégées, par exemple par des groupes protecteurs fixés par des liaisons covalentes labiles dans l'eau ou par des liaisons ioniques directement dissociées en présence d'eau.

Dans un mode de mise en oeuvre particulièrement avantageux de l'utilisation selon l'invention, les molécules (A) sont choisies de préférence parmi les aminoacides et leurs dérivés.

Dans un mode de mise en oeuvre encore plus avantageux de l'utilisation selon l'invention, les molécules (A) comprenant un ou plusieurs groupes amino protégés par des groupes protecteurs sont choisies parmi les dérivés d'aminoacides qui sont décrits dans le brevet US N° 3,392,195, et qui répondent à la formule (I) : 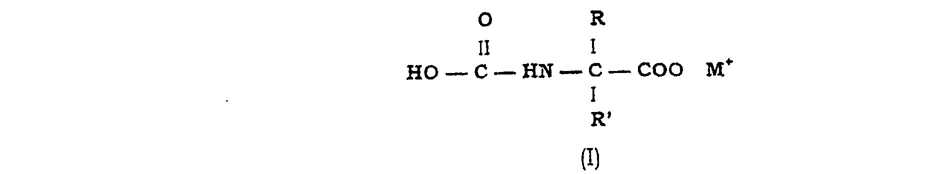 ou à la formule (II) 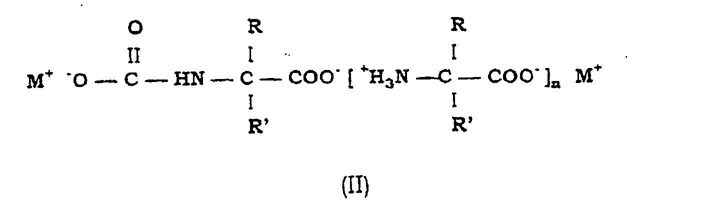 dans lesquelles
R et R' sont choisis indépendamment l'un de l'autre dans le groupe constitué par l'atome d'hydrogène, les groupes aliphatiques et aromatiques,
n est un entier compris entre 1 et 9,
M représente un métal alcalin choisi dans le groupe constitué par le lithium, le sodium et le potassium.

Dans un mode de mise en oeuvre encore plus avantageux de l'utilisation selon l'invention, les molécules (A) comprenant un ou plusieurs groupes amino protégés par des groupes protecteurs sont choisies dans le groupe constitué par le carbonate de glycine sodique et le bicarbonate d'arginine.

Dans un autre mode de mise en oeuvre avantageux de l'utilisation selon l'invention, les molécules (B) sont des monosaccharides.

Dans un mode de mise en oeuvre encore plus avantageux de l'utilisation selon l'invention, les monosaccharides sont choisis dans le groupe constitué par le glucose et le lactose.

Conformément à l'utilisation selon l'invention, les compositions sèches peuvent contenir en outre, au moins un principe actif choisi dans le groupe comprenant : les vitamines, les acides aminés,et protéines, les oligo-éléments, les antibiotiques, les anti-infectieux de synthèse, les antiparasitaires, les facteurs de croissance, les antibactériens, les antifongiques, les antiseptiques, lesdits principes actifs pouvant être une molécule (A) comprenant un ou plusieurs groupes amino protégés par des groupes protecteurs, lesdits groupes protecteurs s'éliminant en présence d'eau, ou une molécule (B).

Conformément à l'utilisation selon l'invention, les compositions sèches peuvent contenir également au moins un autre constituant choisi dans le groupe constitué par les agents de charge, les agents chélatants, les agents anti-mottants, les arômes, les parfums, les liants et les lubrifiants.

Conformément à l'invention, l'agent de charge peut être choisi dans le groupe constitué par l'acide citrique, l'acide tartrique, l'acide ascorbique, l'acide adipique, l'acide malique et l'acide fumarique.

Conformément à l'utilisation selon l'invention, les compositions sèches peuvent contenir également des lipides, sous forme d'acides gras à chaînes moyennes, comprenant de 8 à 14 atomes de carbone, des phospholipides comme la lécithine de soja et des électrolytes comme le sodium, le potassium, le magnésium, le calcium, sous forme de sels inorganiques ou organiques, notamment sous forme de bicarbonates, de chlorures, d'acétates, de propionates et de citrates.

Dans un mode de mise en oeuvre particulièrement avantageux de l'utilisation selon l'invention, on utilise :
- 8 à 20 % (m/m) d'au moins une molécule (A) comprenant un ou plusieurs groupes amino protégés par des groupes protecteurs, lesdits groupes protecteurs s'éliminant en présence d'eau, en association avec
- 40 à 80 % d'au moins une molécule (B) portant un ou plusieurs groupes carbonyle, et éventuellement,
- 5 à 40 % de un ou plusieurs composés choisis dans le groupe constitué par les lipides et les électrolytes.

Dans un mode de mise en oeuvre encore plus avantageux de l'utilisation selon l'invention, on utilise :
- 8 à 20 % (m/m) de carbonate de glycine sodique en association avec
- 40 à 80 % de lactose ou de glucose et
- 5 à 40 % de un ou deux composés choisis dans le groupe constitué par le chlorure de potassium et l'acétate de sodium.

Dans un mode de réalisation particulièrement avantageux de l'invention, les compositions sèches sont utilisées comme médicament à usage vétérinaire, notamment dans la réhydratation des jeunes animaux.

Dans un autre mode de réalisation particulièrement avantageux de l'invention, les compositions sèches sont utilisées, comme produit alimentaire, diététique ou cosmétique.

Une telle utilisation permet d'obtenir des compositions sèches, qui sont protégées de la réaction de Maillard et ne sont donc pas modifiées à l'état sec. Les compositions obtenues par le procédé selon l'invention, permettent de garantir l'intégrité de leurs constituants lors du stockage sous leur forme initiale. La réaction de Maillard n'ayant pas lieu, lesdites compositions présentent l'avantage supplémentaire d'éviter la formation d'eau consécutive à cette réaction. De ce fait, tous les constituants des compositions sèches sont protégés des phénomènes consécutifs à une hydratation (dégradation, mottage...).

De plus, les compositions ainsi obtenues, permettent, lors de leur utilisation, d'éviter les risques d'erreurs ou d'oublis qui peuvent, conduire à des sur-titrages ou à des sous-titrages.

Pour la mise en oeuvre de l'utilisation selon l'invention, toutes les techniques classiques connues de l'homme du métier, permettant d'obtenir des compositions sèches sous forme pulvérulente, granulée ou comprimée, peuvent être utilisées, notamment le mélange mécanique des différents constituants par mélange à sec des composants en poudre, puis éventuellement granulation par voie sèche (frittage, compactage), ou par voie humide (par des liquides non aqueux ou par l'eau en contrôlant la réaction ou par lit d'air fluidisé), et/ou éventuellement compression directe après mélange à sec des composants en poudre ou compression de la composition préalablement granulée.

Les Exemples qui suivent illustrent l'invention sans la limiter.

### EXEMPLE 1

Préparation de compositions sèches pulvérulentes solubles conformément à l'utilisation selon l'invention (N°1) et selon l'état antérieur de la technique (N°2)

| COMPOSANTS | COMPOSITION SECHE | |
|---|---|---|
| | N°1 | N°2 |
| Chlorure de Potassium | 2,65 g | 2,65 g |
| Acétate de Sodium | 4,60 g | 4,60 g |
| Arôme Vanille | 0,10 g | 0,10 g |
| Acide citrique | 8,75 g | 8,75 g |
| Lactose = (B) | 67,90 g | 67,9 g |
| Carbonate de glycine sodique = (A) comprenant un groupe amino protégé par un groupe protecteur selon l'invention | 16,00 g | - |
| Glycine (A) | - | 9,60 g |
| Carbonate de sodium | - | 6,40 g |

On mélange mécaniquement les différents constituants de chaque composition sèche et on les conditionne dans des flacons de verre hermétiquement clos.

### a) test de stabilité à l'état sec :

Les flacons sont conservés dans une étuve à 40°C et ils sont observés régulièrement. Au bout de trois jours, on note un brunissement de la composition sèche N°2 avec une prise en masse. En revanche, avec la composition sèche N°1 on ne note aucune modification de coloration et la composition sèche reste parfaitement fluide. Les observations faites les jours et semaines suivantes montrent une intensification du phénomène pour la composition sèche N°2, ce qui conduit à un noircissement et à une liquéfaction apparaissant au bout de quinze jours et très marqués au bout de 4 semaines. En revanche, aucune modification n'est perceptible pour la composition sèche N°1 selon l'invention.

### b) dosage de la glycine après mise en solution des compositions sèches N°1 et N°2

Après deux semaines de conservation de la composition à l'état sec, on constate après mise en solution de la composition sèche N°2, que la quantité de glycine présente est négligeable pour 100 g de composition sèche. La réaction de Maillard est la principale cause de la disparition de l'aminoacide. La composition sèche N°1 mise en solution au même moment restitue quant-à elle 9,6 g de glycine pour 100 g de composition sèche et 67,9 g de lactose. La préparation est restée stable à l'état sec.

Ces résultats montrent que la composition sèche N°1, composition sèche selon l'invention, est stable à l'état sec, soluble dans l'eau et garantit l'intégrité des constituants.

### EXEMPLE 2

Granulés énergisants solubles effervescents préparés conformément à l'invention

| FORMULE | |
|---|---|
| Acide ascorbique | 12,50 g |
| Nicotinamide | 1,50 g |
| Biotine | 7,50 g |
| Tocophérol | 1000 U.I. |
| Retinol sous forme de propionate | 150 000 U.I. |
| Sulfate de Cuivre | 21 mg |
| Sulfate de Zinc | 71,5 mg |
| Carbonate de glycine sodique = (A) comprenant un groupe amino protégé par un groupe protecteur selon l'invention | 10,00 g |
| Polyvinylpyrrolidone | 5,00 g |
| Glucose = (B) | qsp 100 g |

Cette formulation conditionnée en sachet de 50 g, montre une excellente stabilité entre 4°C et 40°C avec une humidité relative de 60% pendant 6 mois. Aucune modification de l'aspect des granulés, en particulier de la couleur, de l'aspect de la solution (goût et couleur) ou du pouvoir effervescent n'a été notée. Aucune prise en masse, ni aucune variation des teneurs en vitamines n'ont été enregistrées.

### EXEMPLE 3

Comprimés solubles effervescents contenant de l'amoxicilline comme principe actif

| FORMULE | |
|---|---|
| Amoxicilline modifiée * | 1,00 g |
| Acide ascorbique | 0,50 g |
| Citrate de Magnésium | 0,18 g |
| Benzoate de Potassium | 0,04 g |
| Acide citrique | 0,27 g |
| Aspartam | 0,01 g |
| Carbonate de glycine sodique = (A) comprenant un groupe amino protégé par un groupe protecteur selon l'invention | 0,52 g |
| Lactose = (B) | 0,50 g |

| | |
|---|---|
| *groupe amino protégé par réaction avec du carbonate sodique selon le brevet FR 2285127 | |

Comme dans l'exemple 2, les comprimés conditionnés en piluliers de verre ou en blisters d'aluminium n'ont présenté aucun changement d'aspect, principalement de couleur et de goût, ni de modification du pouvoir effervescent. L'activité antimicrobienne de l'amoxicilline n'a pas varié tout au long de l'étude de stabilité.

## Revendications

1. Utilisation d'au moins une molécule (A) comprenant un ou plusieurs groupes amino protégés par des groupes protecteurs, lesdits groupes protecteurs s'éliminant en présence d'eau, en association avec au moins une molécule (B) portant un ou plusieurs groupes carbonyle, pour éviter la réaction de Maillard dans des compositions sèches à l'état sec et solubles en présence d'eau.

2. Utilisation selon la revendication 1, **caractérisée en ce que** les molécules (A) sont choisies de préférence parmi les aminoacides et leurs dérivés.

3. Utilisation selon la revendication 2, **caractérisée en ce que** les molécules (A) comprenant un ou plusieurs groupes amino protégés par des groupes protecteurs sont choisies parmi les dérivés d'aminoacides de formule (I) : 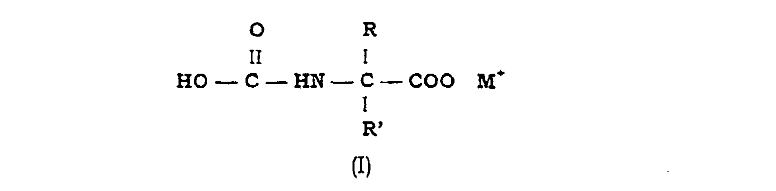 ou de formule (II) 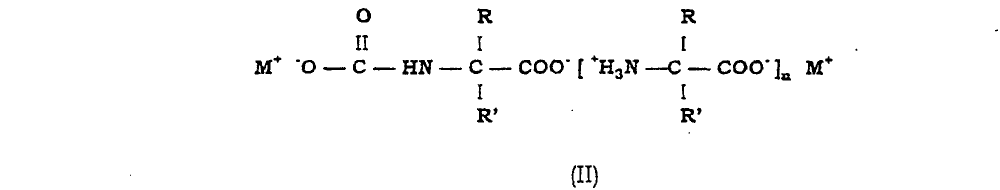 dans lesquelles
R et R' sont choisis indépendamment l'un de l'autre dans le groupe constitué par l'atome d'hydrogène, les groupes aliphatiques et aromatiques,
n est un entier compris entre 1 et 9,
M représente un métal alcalin choisi dans le groupe constitué par le lithium, le sodium et le potassium.

4. Utilisation selon la revendication 3, **caractérisée en ce que** les molécules (A) comprenant un ou plusieurs groupes amino protégés par des groupes protecteurs sont choisies dans le groupe constitué par le carbonate de glycine sodique et le bicarbonate d'arginine.

5. Utilisation selon l'une quelconque des revendications 1 à 4, **caractérisée en ce que** les molécules (B) sont des monosaccharides.

6. Utilisation selon la revendication 5, **caractérisées en ce que** les monosaccharides sont choisis dans le groupe constitué par le glucose et le lactose.

7. Utilisation selon l'une quelconque des revendications 1 à 6, **caractérisée en ce que** les compositions sèches contiennent en outre au moins un principe actif choisi dans le groupe comprenant : les vitamines, les acides aminés et protéines, les oligo-éléments, les antibiotiques, les anti-infectieux de synthèse, les antiparasitaires, les facteurs de croissance, les antibactériens, les antifongiques, les antiseptiques, lesdits principes actifs pouvant être une molécule (A) comprenant un ou plusieurs groupes amino protégés par des groupes protecteurs, lesdits groupes protecteurs s'éliminant en présence d'eau ou une molécule (B).

8. Utilisation selon l'une quelconque des revendications 1 à 7, **caractérisée en ce que** les compositions sèches contiennent en outre au moins un autre constituant choisi dans le groupe constitué par les agents de charge, les agents chélatants, les agents anti-mottants, les arômes, les parfums, les liants et les lubrifiants.

9. Utilisation selon la revendication 8, **caractérisée en ce que** l'agent de charge est choisi dans le groupe constitué par l'acide citrique, l'acide tartrique, l'acide ascorbique, l'acide adipique, l'acide malique et l'acide fumarique.

10. Utilisation selon l'une quelconque des revendications 1 à 9, **caractérisée en ce que** les compositions sèches contiennent également des lipides, sous forme d'acides gras à chaînes moyennes, comprenant de 8 à 14 atomes de carbone, des phospholipides et des électrolytes.

11. Utilisation selon la revendication 10, **caractérisée en ce que** le phospholipide est la lécithine de soja et **en ce que** les électrolytes sont choisis dans le groupe constitué par le sodium, le potassium, le magnésium, le calcium, sous forme de sels inorganiques ou organiques.

12. Utilisation selon la revendication 10 caractérisée en que l'on utilise :
- 8 à 20 % (m/m) d'au moins une molécule (A) comprenant un ou plusieurs groupes amino protégés par des groupes protecteurs, lesdits groupes protecteurs s'éliminant en présence d'eau, en association avec
- 40 à 80 % d'au moins une molécule (B) portant un ou plusieurs groupes carbonyle, et éventuellement,
- 5 à 40 % de un ou plusieurs composés choisis dans le groupe constitué par les lipides et les électrolytes.

13. Utilisation selon la revendication 12, **caractérisée en ce que** l'on utilise :
- 8 à 20 % (m/m) de carbonate de glycine sodique en association avec
- 40 à 80 % de lactose ou de glucose et
- 5 à 40 % de un ou deux composés choisis dans le groupe constitué par le chlorure de potassium et l'acétate de sodium.

14. Utilisation selon l'une quelconque des revendications 1 à 13, **caractérisée en ce que** les compositions sèches sont utilisées pour l'obtention d'un médicament destiné à un usage vétérinaire, notamment dans la réhydratation des jeunes animaux.

15. Utilisation selon l'une quelconque des revendications 1 à 13, **caractérisée en ce que** les compositions sèches sont utilisées, comme produit alimentaire, diététique ou cosmétique.

## Patentansprüche

1. Verwendung wenigstens eines Moleküls (A), das ein oder mehrere durch Schutzgruppen geschützte Aminogruppen umfaßt, wobei diese Schutzgruppen in Gegenwart von Wasser abgespalten werden, zusammen mit wenigstens einem Molekül (B), das ein oder mehrere Carbonylgruppen trägt, zur Vermeidung der Maillard-Reaktion im Trockenzustand in trockenen, in Gegenwart von Wasser löslichen Zusammensetzungen.

2. Verwendung nach Anspruch 1, **dadurch gekennzeichnet, daß** die Moleküle (A) vorzugsweise aus Aminosäuren und ihren Derivaten ausgewählt sind.

3. Verwendung nach Anspruch 2, **dadurch gekennzeichnet, daß** die Moleküle (A), die ein oder mehreren durch Schutzgruppen geschützte Aminogruppen umfassen, ausgewählt sind aus den Aminosäurederivaten der Formel (I) : 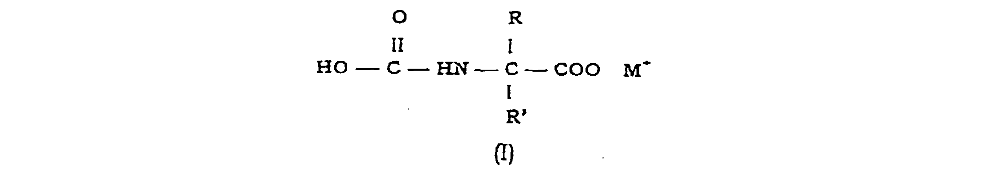 oder Formel (II) 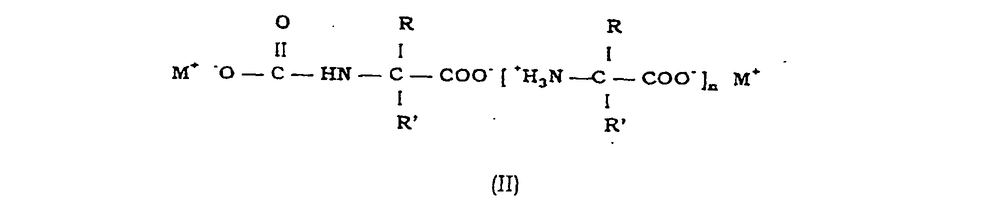 worin
R und R' unabhängig voneinander ausgewählt sind aus der Gruppe bestehend aus Wasserstoffatom, aliphatischen und aromatischen Gruppen,
n eine ganze Zahl zwischen 1 und 9 ist,
M ein Alkalimetall bedeutet, das aus der Gruppe ausgewählt ist, die aus Lithium, Natrium und Kalium besteht.

4. Verwendung nach Anspruch 3, **dadurch gekennzeichnet, daß** die Moleküle (A), die ein oder mehrere durch Schutzgruppen geschützte Aminogruppen umfassen, ausgewählt sind aus der Gruppe bestehend aus Natrium-Glycincarbonat und Arginin-Bicarbonat.

5. Verwendung nach irgendeinem der Ansprüche 1 bis 4, **dadurch gekennzeichnet, daß** die Moleküle (B) Monosaccharide sind.

6. Verwendung nach Anspruch 5, **dadurch gekennzeichnet, daß** die Monosaccharide ausgewählt sind aus der Gruppe bestehend aus Glucose und Lactose.

7. Verwendung nach irgendeinem der Ansprüche 1 bis 6, **dadurch gekennzeichnet, daß** die trockenen Zusammensetzungen ferner wenigstens einen Wirkstoff enthalten, der ausgewählt ist aus der Gruppe umfassend: Vitamine, Aminosäuren und Proteine, Oligoelemente, Antibiotika, synthetische infektionshemmende Mittel, Antiparasitika, Wachstumsfaktoren, antibakterielle Mittel, Fungizide, Antiseptika, wobei es sich bei diesen Wirkstoffen um ein Molekül (A), das ein oder mehrere durch Schutzgruppen geschützte Aminogruppen umfaßt, wobei diese Schutzgruppen in Gegenwart von Wasser abgespalten werden, oder ein Molekül (B) handeln kann.

8. Verwendung nach irgendeinem der Ansprüche 1 bis 7, **dadurch gekennzeichnet, daß** die trockenen Zusammensetzungen ferner wenigstens einen weiteren Bestandteil enthalten, der ausgewählt ist aus der Gruppe bestehend aus Zusatzstoffen, Komplexbildnern, Antibackmitteln, Aromastoffen, Duftstoffen, Bindemitteln und Gleitmitteln.

9. Verwendung nach Anspruch 8, **dadurch gekennzeichnet, daß** der Zusatzstoff ausgewählt ist aus der Gruppe bestehend aus Zitronensäure, Weinsäure, Ascorbinsäure, Adipinsäure, Äpfelsäure und Fumarsäure.

10. Verwendung nach irgendeinem der Ansprüche 1 bis 9, **dadurch gekennzeichnet, daß** die trockenen Zusammensetzungen ferner Lipide, in Form von Fettsäuren mittlerer Kettenlänge, die 8 bis 14 Kohlenstoffatome umfassen, Phospholipide und Elektrolyte enthalten.

11. Verwendung nach Anspruch 10, **dadurch gekennzeichnet, daß** das Phospholipid Sojalecithin ist und daß die Elektrolyte ausgewählt sind aus der Gruppe bestehend aus Natrium, Kalium, Magnesium und Calcium in Form anorganischer oder organischer Salze.

12. Verwendung nach Anspruch 10, **dadurch gekennzeichnet, daß** man verwendet:
- 8 bis 20% (w/w) wenigstens eines Moleküls (A), das ein oder mehrere durch Schutzgruppen geschützte Aminogruppen umfaßt, wobei die Schutzgruppen in Gegenwart von Wasser abgespalten werden, zusammen mit
- 40 bis 80% wenigstens eines Moleküls (B), das ein oder mehrere Carbonylgruppen trägt, und gegebenenfalls
- 5 bis 40% einer oder mehrerer Verbindungen, die ausgewählt sind aus der Gruppe bestehend aus Lipiden und Elektrolyten.

13. Verwendung nach Anspruch 12, **dadurch gekennzeichnet, daß** man verwendet:
- 8 bis 20% (w/w) Natrium-Glycincarbonat, zusammen mit
- 40 bis 80% Lactose oder Glucose und
- 5 bis 40% einer oder zweier Verbindungen, die ausgewählt sind aus der Gruppe bestehend aus Kaliumchlorid und Natriumacetat.

14. Verwendung nach irgendeinem der Ansprüche 1 bis 13, **dadurch gekennzeichnet, daß** die trockenen Zusammensetzungen zur Herstellung eines Medikaments zur veterinärmedizinischen Verwendung, insbesondere zur Rehydratation von Jungtieren, verwendet werden.

15. Verwendung nach irgendeinem der Ansprüche 1 bis 13, **dadurch gekennzeichnet, daß** die trockenen Zusammensetzungen als Nahrungsmittel, diätetisches Mittel oder kosmetisches Mittel verwendet werden.

## Claims

1. Use of at least one molecule (A) comprising one or more amino groups protected by protecting groups, said protecting groups being eliminated in the presence of water, combined with at least one molecule (B) carrying one or more carbonyl groups, to prevent the Maillard reaction in the dry state in dry compositions which are soluble in water.

2. Use according to claim 1, **characterised in that** the molecules (A) are preferably selected from among the amino acids and derivatives thereof.

3. Use according to claim 2, **characterised in that** the molecules (A) comprising one or more amino groups protected by protecting groups are selected from the amino acid derivatives of formula (I): 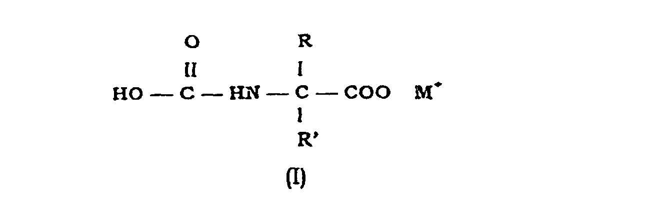 or formula (II) 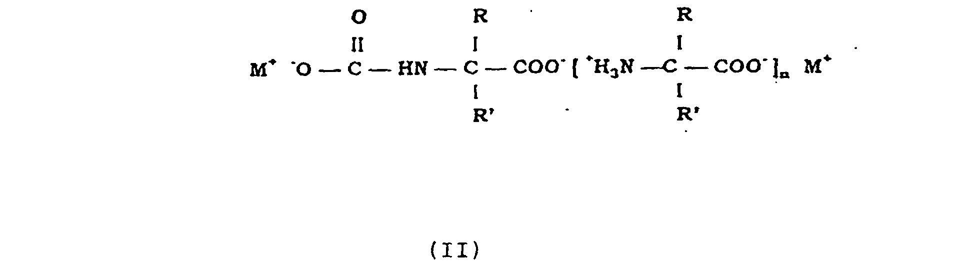 wherein
R and R' are selected, independently of one another, from the group consisting of a hydrogen atom and aliphatic and aromatic groups,
n is an integer between 1 and 9,
M denotes an alkali metal selected from the group consisting of lithium, sodium and potassium.

4. Use according to claim 3, **characterised in that** the molecules (A) containing one or more amino groups protected by protecting groups are selected from the group consisting of sodium glycine carbonate and arginine bicarbonate.

5. Use according to any one of claims 1 to 4, **characterised in that** the molecules (B) are monosaccharides.

6. Use according to claim 5, **characterised in that** the monosaccharides are selected from the group consisting of glucose and lactose.

7. Use according to any one of claims 1 to 6, **characterised in that** the dry compositions further contain at least one active principle selected from the group comprising: vitamins, amino acids and proteins, oligo-elements, antibiotics, synthetic anti-infectious agents, antiparasitic agents, growth factors, antibacterial agents, antifungal agents, antiseptics, where said active principles may be a molecule (A) comprising one or more amino groups protected by protecting groups, said protecting groups being eliminated in the presence of water, or a molecule (B).

8. Use according to any one of claims 1 to 7, **characterised in that** the dry compositions further contain at least one other ingredient selected from the group consisting of fillers, chelating agents, anticaking agents, flavourings, perfumes, binders and lubricants.

9. Use according to claim 8, **characterised in that** the filler is selected from the group consisting of citric acid, tartaric acid, ascorbic acid, adipic acid, malic acid and fumaric acid.

10. Use according to any one of claims 1 to 9, **characterised in that** the dry compositions also contain lipids, in the form of medium-chain fatty acids comprising 8 to 14 carbon atoms, phospholipids and electrolytes.

11. Use according to claim 10, **characterised in that** the phospholipid is soya lecithin and **in that** the electrolytes are selected from the group consisting of sodium, potassium, magnesium and calcium, in the form of organic or inorganic salts.

12. Use according to claim 10, **characterised in that**
- 8 to 20% (w/w) of at least one molecule (A) is used, containing one or more amino groups protected by protecting groups, said protecting groups being eliminated in the presence of water, combined with
- 40 to 80% of at least one molecule (B) carrying one or more carbonyl groups, and optionally
- 5 to 40% of one or more compounds selected from the group consisting of lipids and electrolytes.

13. Use according to claim 12, **characterised in that**
- 8 to 20% (w/w) of sodium glycine carbonate are used, combined with
- 40 to 80% of lactose or glucose and
- 5 to 40% of one or two compounds selected from the group consisting of potassium chloride and sodium acetate.

14. Use according to any one of claims 1 to 13, **characterised in that** the dry compositions are used to obtain a medicament intended for veterinary use, particularly in the rehydration of young animals.

15. Use according to any one of claims 1 to 13, **characterised in that** the dry compositions are used as food products, dietetic products or cosmetic products.
